(19) Europäisches Patentamt
European Patent Office
Office européen des brevets

(11) Publication number: 0014017 A1

# (12) EUROPEAN PATENT APPLICATION

(21) Application number: 80200045.5

(22) Date of filing: 18.01.80

(51) Int. Cl.³: C 07 C 93/08, A 61 K 31/13

(30) Priority: 26.01.79 DE 2903018

(43) Date of publication of application: 06.08.80 Bulletin 80/16

(84) Designated Contracting States: AT BE CH DE FR GB IT LU NL SE

(71) Applicant: Chem. Pharmaz. Fabrik Dr. Hermann Thiemann GmbH, Kirchstrasse 12 - 16, D-4670 Luenen/Westf. (DE)

(72) Inventor: Cambanis, Anton, Laakstrasse 42b, D-4670 Luenen/Westf. (DE)
Inventor: Mayer, Dieter, Beethovenstrasse 2, D-4712 Werne (DE)
Inventor: Bohn, Helmut, Margarittenring 4a, D-4710 Luedinghausen (DE)
Inventor: Magda, Stephan, Gellerstrasse 24, D-3000 Hannover (DE)
Inventor: Schmitz, Dieter, Karlstrasse 8, D-4407 Emsdetten (DE)

(74) Representative: Hermans, Franciscus G.M., Postbus 20, NL-5340 BH Oss (NL)

## (54) 1-(Omega-aminoalkoxy)-3,3,5-trimethylcyclohexanes, processes for their preparation and pharmaceutical compositions containing same.

(57) The invention relates to novel 1-($\omega$-aminoalkoxy)-3,3,5-trimethylcyclohexanes and their salts of the general formula:

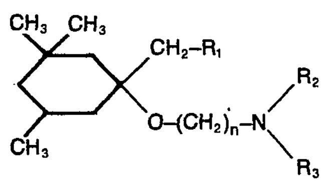

I

where

$R_1$ denotes a straight chain or branched alkyl, or an aryl or aralkyl-residue, which may be substituted by alkyl residues or halogen atoms;

$R_2$ and $R_3$ denote a straight chain or branched alkyl residue and

n represents 2, 3, or 4,

having valuable cardiovascular properties.

EP 0 014 017 A1

1-(ω-AMINOALKOXY)-3,3,5-TRIMETHYLCYCLOHEXANES, PROCESSES FOR THEIR PREPARATION AND PHARMACEUTICAL COMPOSITIONS CONTAINING SAME.

The invention relates to novel 1-(ω-aminoalkoxy)-3,3,5-trimethylcyclohexanes and their salts, to processes for their preparation and to pharmaceutical compositions containing same.

1-(ω-aminoalkoxy)-3,3,5-trimethylcyclohexanes, which possess an additional (unsubstituted) phenyl-residue in the 1-position, are known from West German Patent Specification 1 229 521. These known compounds are described to have a blood pressure increasing effect.

It is an object of the invention to provide novel active substances, which promote blood circulation without having marked hypertensive properties.

It is a further and more general object of the present invention to provide novel substances which are useful in the prophylaxis and/or treatment of cardiovascular disorders.(e.g. angina pectoris, arrhythmia).

Surprisingly, 1-(ω-aminoalkoxy)-3,3,5-trimethyl-cyclohexanes of the general formula:

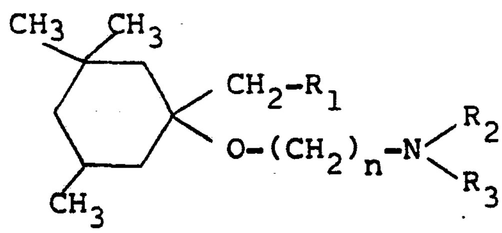

I

and their salts with physiologically acceptable acids, where

$R_1$ denotes a straight chain or branched alkyl, or an aryl or aralkyl residue, which may be substituted by alkyl residues or halogen atoms;

$R_2$ and $R_3$ denote a straight chain or branched alkyl residue and

n represents 2, 3 or 4,

were found to increase coronary and peripheral blood flow and improve the refractory period, in combination with a positively inotropic effect on heart muscle and a moderate lowering of blood pressure.

This biological profile of the present compounds is based on test results obtained in in-vivo experiments (anaesthetized dogs) as well as in in-vitro experiments (with isolated heart or atrium of guinea pigs).

In view of the very close structural similarity of the compounds according to the invention and those known from the German Specification 1 229 521, this biological profile, that is completely different from the said known compounds, is the more surprising.

The compounds of formula I are prepared by methods in actual use or described in the literature. A very convenient method consists of the reaction of a compound of the formula:

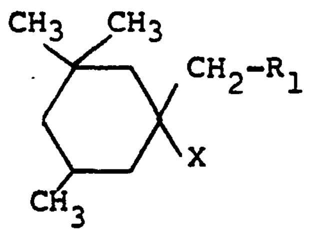

II

with the compound

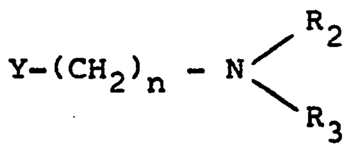

III

or an acid addition salt thereof, in which $R_1$, $R_2$, $R_3$ and n have the meanings indicated before and X and Y are hydroxy or halogen provided that where X is hydroxy, Y is halogen and where X is halogen, Y is hydroxy.

For example, one method is illustrated by the following reaction sequence:

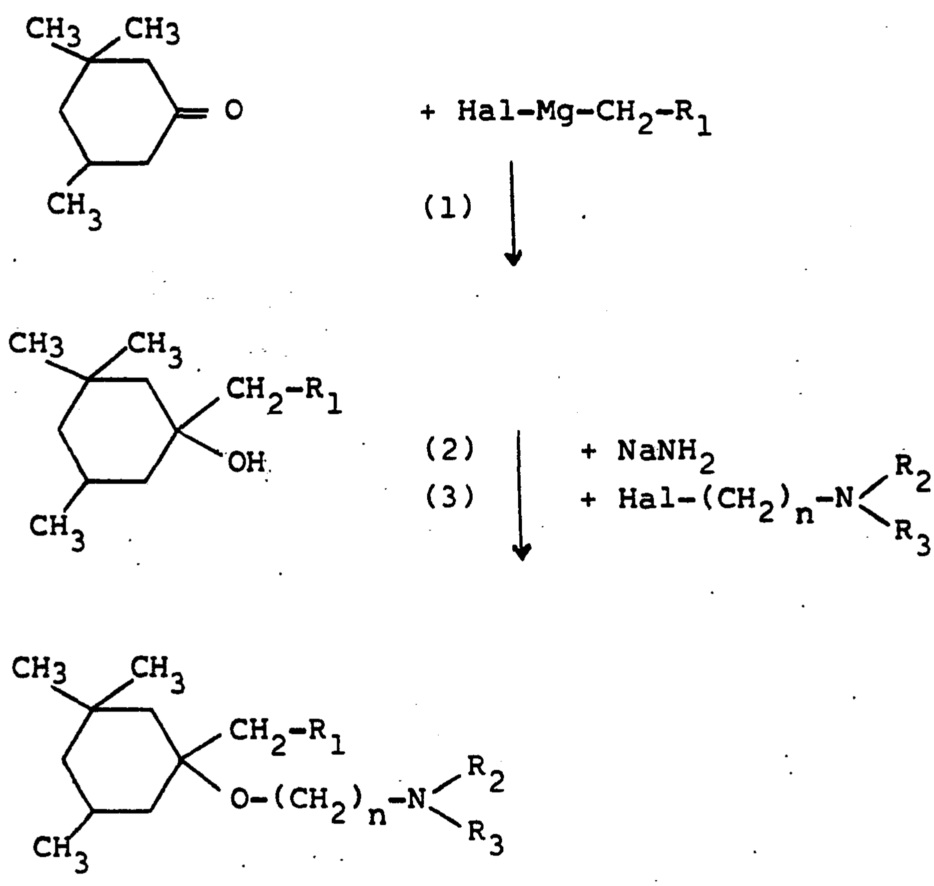

Step 1: For example, the Grignard compound is prepared from the appropriate halogen derivative and magnesium in anhydrous ether and it is reacted with the trimethylated cyclohexanone in anhydrous ether at about 50 °C. The complex formed is decomposed with ammonium chloride and the desired compound is obtained from the organic phase.

Step 2: For example, a suspension of finely pulverised sodium amide in anhydrous benzene is added, with vigorous stirring, to the trimethylated cyclohexan-1-ol, substituted in the 1-position, in anhydrous benzene. The mixture is then heated under reflux.

Step 3: For example, a solution of the alkylaminoalkyl halide in anhydrous benzene is added to the reaction mixture of Step (2) and the whole is heated under reflux. The compound according to the invention is obtained from the reaction residue.

The compounds can also be prepared by halogenating the cyclohexan-1-ol, substituted in the 1-position, by known methods and reacting the halo-derivative of the general formula:

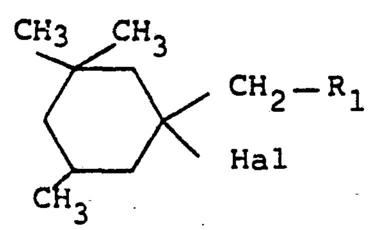

with an amino-alcohol of the general formula:

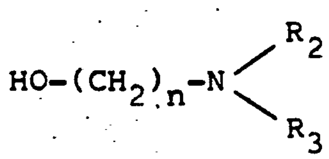

to form the compounds according to the invention.

For the preparation of the acid addition salts, the free bases according to the invention are usually reacted with the acid desired in the usual way.

The compounds of the invention comprise racemates and optically active isomers as well. The latter compounds can be separated from the racemate I in the usual manner. It is also possible, however, to prepare directly the optically active isomers I by using optically active starting material in the preparation methods described before. Examples of suitable acid addition salts according to the invention are hydrochlorides, hydrobromides, nitrates, sulphates and phosphates as well as salts with organic acids such as tartaric acid, maleic acid and fumaric acid. Hydrogen fumarates are especially preferred.

The compounds according to the invention can be processed to customary liquid or solid pharmaceutical preparations, for example, to sugar-coated pills, tablets, suppositories and solutions, also for injection. The customary excipients and diluents are used for this purpose.

The oral single dose ranges from 1 to 60 mg/kg of body weight, the oral daily dose ranges from 3 to 180 mg/kg of body weight. The parenteral single dose ranges from 0.1 to 15 mg/kg of body weight and the parenteral daily dose is the threefold quantity of this single dose.

The residue $R_1$ of the compounds according to the general formula I is preferably alkyl (1-6 C), such as methyl, ethyl, propyl, isopropyl, butyl, sec. butyl, tert. butyl, isobutyl, methylbutyl and n-pentyl, or

aryl or aryl (1-4 C) alkyl, such as phenyl, benzyl, phenylethyl, phenylpropyl, naphthyl and naphthylmethyl.

Preferred $R_1$ residues are isobutyl, phenyl and benzyl.

The aryl residues, particularly the phenyl residue, may be substituted by one or more halogen, viz. chlorine, bromine, iodine and especially fluorine, or by alkyl (1-4 C), especially methyl or ethyl. The para position is the preferred position of the substituent.

Examples of the residues $R_2$ and $R_3$ are methyl, ethyl, propyl, isopropyl, butyl and hexyl. $R_2$ and $R_3$ can be either different or identical residues. Preferably, $R_2$ and $R_3$ are both methyl or both ethyl.

The value of n is preferably 2 or 3.

From a view point of improved blood-flow properties, in combination with a positively inotropic effect the following compounds are preferred:

1-(2-phenylethyl)-1-(3-diethylaminopropoxy)-3,3,5-trimethylcyclohexane and

1-benzyl-1-(3-diethylaminopropoxy)-3,3,5-trimethylcyclohexane

as well as acid addition salts thereof.

These compounds can eminently be used as coronary and peripheral vasodilators.

From a view point of an improved refractory period of the heart in combination with a positively inotropic effect the following compounds are preferred:

1-(3-methylbutyl)-1-(2-dimethylamino-ethoxy)-3,3,5-trimethylcyclohexane and

1-(p-fluorobenzyl)-1-(2-dimethylamino-ethoxy)-3,3,5-trimethylcyclohexane,
as well as acid addition salts thereof.

These compounds may preferably be used in the treatment of arrhythmia.

## Example 1

Preparation of 1-(2-phenylethyl)-1-(3-diethylamino-propoxy)-3,3,5-trimethylcyclohexane hydrogen fumarate.

(a) 0.1 molecule of 1-(2-phenylethyl)-1-hydroxy-3,3,5-trimethylcyclohexane is dissolved in 30 ml of anhydrous benzene and added dropwise, with vigorous stirring, to a suspension of 0.1 molecule of finely pulverised sodium amide in 30 ml of anhydrous benzene during 30 minutes. The reaction mixture then is heated under reflux for 2 hours, cooled to 35-40 °C and a solution of 0.11 molecule of diethylaminopropyl-1-chloride in 20 ml of anhydrous benzene is added. The reaction mixture is heated under reflux for a further 6 hours, cooled and washed 3 times with 50 ml of water. The organic phase is boiled down and the oily residue fractionated in vacuo. 1-(2-phenylethyl)-1-(3'-diethylamino-n-propoxy)-3,3,5-trimethylcyclohexane is obtained.
Yield: 82%; $n_D^{20}$ = 1.4968; b.p.: 156-158 °C/0.1 torr.

(b) 0.1 molecule of 1-(2-phenylethyl)-1-(3'-diethylamino-n-propoxy)-3,3,5-trimethylcyclohexane is dissolved in 50 ml of ethyl alcohol and added to 0.1 molecule of fumaric acid, dissolved in 50 ml of hot ethyl alcohol. The solution is concentrated and the salt precipitated with diethyl ether, being cooled with ice.

1-(2-phenylethyl)-1-(3'-diethylamino-n-propoxy)-3,3,5-trimethylcyclohexane hydrogen fumarate is obtained. Yield: 86%; m.p.: 128-130 °C.
The following compounds according to the invention and their hydrogen fumarates are prepared in an analogous manner:

Table

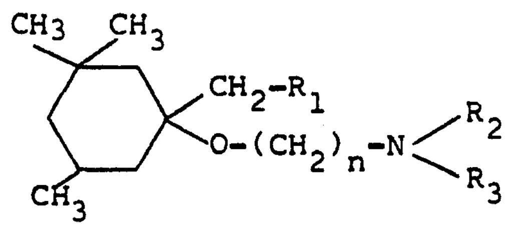

| Example | n | $R_1$ | $R_2$, $R_3$ | b.p. (°C/torr.) | $n_D^{20}$ | m.p. (°C) hydrogen fumarate |
|---|---|---|---|---|---|---|
| 2 | 2 | phenyl | methyl | 126-128/0.08 | 1.5052 | 111-112 |
| 3 | 2 | phenyl | ethyl | 140/0.08 | 1.5014 | 120-121 |
| 4 | 2 | benzyl | methyl | 138-141/0.08 | 1.5004 | 121-122 |
| 5 | 2 | benzyl | ethyl | 140-142/0.1 | 1.4977 | 110-112 |
| 6 | 3 | benzyl | methyl | 150-155/0.1 | 1.4995 | 129-131 |
| 7 | 3 | phenyl | methyl | 134-136/0.08 | 1.5038 | 131-133 |
| 8 | 2 | isobutyl | methyl | 108/0.14 | 1.4552 | 101-103 |
| 9 | 2 | isobutyl | ethyl | 127/0.18 | 1.4563 | 68-69 |
| 10 | 3 | isobutyl | methyl | 115/0.08 | 1.4575 | 120-121 |
| 11 | 3 | phenyl | ethyl | 144-146/0.1 | 1.5008 | 95-97 |
| 12 | 2 | p-fluoro phenyl | methyl | 131-134/0.1 | 1.4962 | 147-148 |
| 13 | 2 | p-fluoro phenyl | ethyl | 158/0.2 | 1.4938 | 129-131 |
| 14 | 3 | p-fluoro phenyl | methyl | 148-150/0.1 | 1.4948 | 79-80 |
| 15 | 2 | p-methyl phenyl | methyl | 135-136/0.12 | 1.5054 | 140-141 |
| 16 | 2 | p-methyl phenyl | ethyl | 140-142/0.11 | 1.5028 | 136-137 |
| 17 | 3 | p-methyl phenyl | methyl | 143-148/0.1 | 1.5043 | 133-135 |

## CLAIMS

1. 1-(ω-aminoalkoxy)-3,3,5-trimethylcyclohexanes of the general formula:

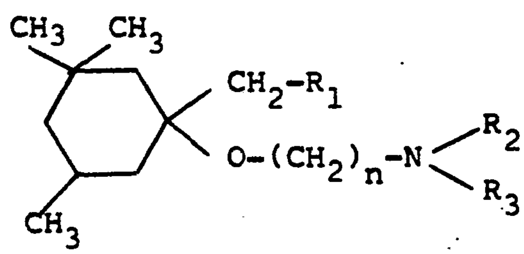

I

and their salts with physiologically acceptable acids,

where

$R_1$ represents a straight or branched chain alkyl residue or an aryl or aralkyl residue, the aryl moiety being optionally substituted by (1-4 C) alkyl or halogen,

$R_2$ and $R_3$ both represent a straight chain or branched (1-6 C) alkyl residue and

n has the value 2, 3 or 4.

2. 1-(2-phenethyl)-1-(3-diethylaminopropoxy)-3,3,5-trimethylcyclohexane and acid addition salts thereof.

3. 1-benzyl-1-(3-diethylaminopropoxy)-3,3,5-trimethylcyclohexane and acid addition salts thereof.

4. 1-(3-methylbutyl)-1-(2-dimethylamino-ethoxy)-3,3,5-trimethylcyclohexane and acid addition salts thereof.

5. 1-(p-fluorobenzyl)-1-(2-dimethylamino-ethoxy)-3,3,5-trimethylcyclohexane and acid addition salts thereof.

6. Process for the preparation of the compounds of claim 1, characterized in that a compound of the general formula:

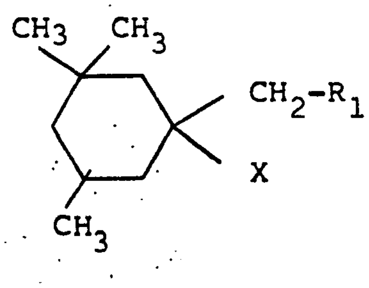

where X denotes OH or halogen, is reacted with a compound of the general formula:

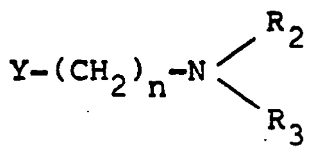

or an acid addition salt thereof,
where Y is halogen or OH, X being OH if Y is halogen or vice versa, and where n, $R_1$, $R_2$ and $R_3$ have the meanings given in claim 1, after which the 1-(ω-aminoalkoxy)-3,3,5-trimethylcyclohexane thus obtained is optionally converted into an acid addition salt thereof.

7. A pharmaceutical preparation containing a compound of claim 1 and a pharmaceutically acceptable carrier or diluent.

European Patent Office

# EUROPEAN SEARCH REPORT

0014017

Application number
EP 80 20 0045

| Category | Citation of document with indication, where appropriate, of relevant passages | Relevant to claim | CLASSIFICATION OF THE APPLICATION (Int. Cl. 3) |
|---|---|---|---|
| | FR - A - 2 361 337 (CHEM. PHARM. FABRIK DR. HERMANN THEMANN)<br>* Claims 1,6,7 *<br>-- | 1,6,7 | C 07 C 93/08<br>A 61 K 31/13 |
| D | DE - B - 1 229 521 (TROPONWERKE DINKLAGE)<br>* Column 1, lines 1-29 *<br>-- | 1,7 | |
| A | CH - A - 388 303 (BOEHRINGER)<br>* Page 1 *<br>-- | 1,7 | TECHNICAL FIELDS SEARCHED (Int.Cl. 3)<br>C 07 C 93/08<br>A 61 K 31/13 |
| A | CH - A - 388 939 (BOEHRINGER)<br>* Page 1 *<br>---- | 1,7 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 24-04-1980 | MOREAU |